# EUROPEAN PATENT SPECIFICATION

(11) **EP 1 362 910 B1**
(45) Date of publication and mention of the grant of the patent: **17.02.2010**
(21) Application number: 02700643.6
(22) Date of filing: 20.02.2002
(51) Int. Cl.: C12N 15/09, C12P 21/02, C12Q 1/02, G01N 33/48, C07K 14/42, C12N 15/10, G01N 33/80

(54) **LECTINS FOR ANALYZING SUGAR CHAINS AND METHOD OF USING THE SAME**
LECTINE ZUR ANALYSE VON ZUCKERKETTEN UND VERFAHREN ZU IHRER VERWENDUNG
LECTINES PERMETTANT D'ANALYSER DES CHAINES GLUCIDIQUES ET LEUR PROCEDE D'UTILISATION

(30) Priority: 20.02.2001 JP 2001044221
(43) Date of publication of application: 19.11.2003
(73) Proprietor: Summit Glycoresearch Corporation, Tokyo (JP)
(72) Inventor: IRIMURA, Tatsuro, Setagaya-ku, Tokyo 154-0015 (JP); MATSUMOTO, Mariko, Kawasaki-shi, Kanagawa 211-0954 (JP); YIM, Mijung, Seoul 151-818 (KR); ONO, Takashi, Nakakoma-gun, Yamanashi 400-0316 (JP)
(74) Representative: Baldock, Sharon Claire
(86) International application number: PCT/JP2002/001503
(87) International publication number: WO 2002/066634

(56) References cited:
- WO-A-97/49989
- YAMAZAKI T ET AL: "Fractionation of mouse cytotoxic T cells by use of lectins." CARBOHYDRATE RESEARCH. 16 AUG 1983, vol. 120, 16 August 1983 (1983-08-16), pages 269-281, XP002291082 ISSN: 0008-6215
- KIMURA A ET AL: "Selective affinity fractionation of murine cytotoxic T lymphocytes (CTL). Unique lectin specific binding of the CTL associated surface glycoprotein, T 145" JOURNAL OF EXPERIMENTAL MEDICINE 1979 UNITED STATES, vol. 149, no. 2, 1979, pages 473-484, XP002291083
- SUGIMOTO N ET AL: "Development of small peptides recognizing a monosaccharide by combinatorial chemistry" CHEMICAL COMMUNICATIONS 07 DEC 2000 UNITED KINGDOM, vol. 6, no. 23, 7 December 2000 (2000-12-07), pages 2295-2296, XP002291084 ISSN: 1359-7345
- MATSUI TAEI ET AL: "Comparative study of blood group-recognizing lectins toward ABO blood group antigens on neoglycoproteins, glycoproteins and complex-type oligosaccharides" BIOCHIMICA ET BIOPHYSICA ACTA, vol. 1525, no. 1-2, 16 February 2001 (2001-02-16), pages 50-57, XP002291109 ISSN: 0006-3002
- LIS, HALINA ET AL: "Lectins: Carbohydrate-Specific Proteins that Mediate Cellular Recognition" CHEMICAL REVIEWS (WASHINGTON, D. C.) , 98 (2), 637 -674 CODEN: CHREAY; ISSN: 0009-2665, 1998, XP002291086
- MI-JUNG YIM ET AL.: 'Generation of libraries of genetically engineered plant lectins with diverse carbohydrate specificities' 5TH ANNUAL CONFERENCE OF THE SOCIETY FOR GLYCOBIOLOGY vol. 10, no. 10, 2000, pages 1094 - 1095, XP002951055
- KAZUO YAMAMOTO ET AL.: 'Cyborg lectins: novel leguminous lectins with unique specificities' J. BIOCHEM. vol. 127, 2000, pages 137 - 142, XP002951056
- ANNE IMBERTY ET AL.: 'An unusual carbohydrate binding site revealed by the structure of two maackia amurensis lectins complexed with sialic acid-containing oligosaccharides' J. BIOL. CHEM. vol. 275, no. 23, June 2000, pages 17541 - 17548, XP002951057
- KAZUO YAMAMOTO ET AL.: 'Sialic acid-binding motif of maackia amurensis lectins' J. BIOCHEM. vol. 121, no. 4, April 1997, pages 756 - 761, XP002951058
- MIJUNG YIM ET AL.: 'Mutated plant lectin library useful to identify different cells' PROC. NATL. ACAD. SCI. USA vol. 98, no. 5, February 2001, pages 2222 - 2225, XP002951059

## Description

### Technical Field of the Invention

The present invention relates to a technique of analyzing cell surface sugar chains or the like by using lectins. In particular, the present invention relates to a sugar chains analyzing library and sub-library comprising lectins that can analyze sugar chains, and relates to a method of analyzing sugar chains by using the library and sub-library for analyzing sugar chains. Further, the present invention relates to a library and sub-library of lectins for use in identifying cells, which can easily and accurately identify the type of particular cells. The present invention also relates to a method of identifying the type of cells by using the library and sub-library for identifying cells.

### Background Art

In recent years, it has been said that if the code recorded in sugar chains could be decoded, new horizons of biology would be opened. As a tool for analyzing various sugar chains or as a molecular candidate decoding such a code in the living body, carbohydrates-binding proteins (lectins) can be mentioned. However, each individual lectin cannot give macroscopic information for appropriately reflecting the variety of sugar chains.

That is, it can duly be expected that if a number of different types of lectins could be obtained, sufficient enough to adequately correspond to the variation of sugar chains, they can constitute a useful tool for analyzing various types of sugar chains.
Yamazaki, T et al., Carbohydrate Research, Vol. 120, pages 269-281, discloses a panel/library of over 20 lectins covalently attached to Sepharose and their use in fractionating cytotoxic T cells.
Kimura, A et al., Journal of Experimental Medicine, 1979, Vol. 149, No. 2, pages 473-484, discloses fractioning of T cells by a panel/library of 10 lectins.
Sugimoto, N et al., Chemical Communications, 2000, Vol. 6, No. 23, pages 2295-2296, discloses selection of monosaccharide binding peptides (lectin fragments) from a library.
Matsui Taei et al., Biochimica et Biophysica Acta, Vol. 1525, No. 1-2, 2001, pages 50-57, discloses a panel/library of lectins used in ELISA to differentiate blood group antigens.
WO 97/49989 A, discloses a patterned biotinylated lectin array comprising 8 lectins suitable for the differentiation of glycoproteins.
Lis, Halina et al., Chemical Reviews, 1998 (2), pages 637-674, reviews the structural diversity of lectins.
Mi-Jung, Yim et al., 5TH Annual Conference of the Society for Glycobiology, Vol. 10, No. 10, 2000, pages 1094-1095, discloses lectin variants/libraries with mutated carbohydrate-recognition domain of MAH.
Kazuo, Yamamoto et al., J. Biochem, Vol. 127, 2000, pages 137-142, discloses a phage display library of BPA lectins.
Imberty, Anne et al., J. Biochem, Vol. 275, No. 23, 2000, pages 17541-17548, discloses the 3D structure of the carbohydrate binding loops of MAL and MAH.
Kazuo, Yamamoto et al., J. Biochem, Vol. 121, No. 4, 1997, pages 756-761, discloses K105 and D135 as critical residues of MAH in carbohydrate binding.

### Disclosure of Invention

In view of the problem described above, a lectin sub-library is constructed in the present invention by selecting lectins necessary for analyzing the sugar chains disclosed in the claims, from a lectin library comprising plural types of lectins as disclosed in the claims. By using the lectin sub-library thus constructed, cells having sugar chains are identified. Further, a method of display capable of easily identifying cells is disclosed herein. That is, sugar chains are hardly specified by using one type of lectin, but they can be identified, by using a lectin sub-library comprising a combination of different types of lectins. Further, even in the case that the sugar chains structure cannot be fully specified in details, the lectin sub-library can still be used as a tool to identify and to make a comparison of sugar chains wherein a difference in cells (including a difference in type and a difference between normality and abnormality) represented by a difference of sugar chains, or a difference in the glycosylation modification of proteins can be recognized, or a substance carrying sugar chains as markers can be discriminated. On the other hand, when the relationship is clear between a lectin sub-library and cells, glycosylation modification of glycoproteins, or sugar chains markers, the difference in cells, the difference of glycosylation modification of glycoproteins, sugar chains markers can be identified from the relationship, without elucidating the actual structure of sugar chains.

In particular, the present invention provides:
(1) A method of analyzing sugar chains, which comprises using a cell-identifying lectin sub-library comprising a series of lectins as disclosed in the claims each having a modification in a predetermined site in the lectin, or plural types of lectins obtained by pannings with cells or pseudo-cells having sugar chains as disclosed in the claims.

Preferably the lectin library is formed as an array of lectins on a solid carrier wherein lectin-immobilizing regions each contain a lectin selected to identify sugar chains, arranged in a suitable order for identifying sugar chains. The lectin-immobilizing region may be a well or a simple spot, e.g. a region not particularly delimitated by an external structure, or may be a part or place where lectins can be immobilized. The solid carrier may be in a form of chip or plate or any other forms of solid material capable of forming the lectin-immobilizing regions thereon.

Preferably, the lectin-immobilizing regions are arranged in a suitable order for identifying/analysing sugar chains, and each region contains a lectin selected for identifying/analysing sugar chains.

Generally, the term "lectin" is a generic name of proteins specifically recognizing and binding to sugar chains, which are roughly divided into animal lectins and plant lectins. Among the plant lectins, leguminous lectins constitute a large lectin family. The lectin is composed of a dimer or tetramer of subunits each having a molecular weight of 30, 000. Each subunit has one sugar chains-recognizing site. Lectin is sometime defined in a narrow sense as "a multivalent, carbohydrates binding protein excluding enzymes or antibodies, or defined as a glycoprotein", or can be defined as "a carbohydrates recognizing protein binding and cross-linking to carbohydrates" in a broad sense, and has thus the ability to identify and analyze many types of a carbohydrates substantially. Particularly, the variation of carbohydrates may reflect the variation of cells having such carbohydrates on the surface. Therefore, cells can be identified by identifying/analyzing their cell surface carbohydrates.

Unlike antibodies, lectins are found in every organ and tissue in almost all living things. Further, the structures of lectins are varied, while the structures of antibodies are similar.

In the present invention, MAH lectins are used.

Lectins having specific binding properties to certain sugar chains disclosed in the claims may be selected and used without any modification as a lectin library, or mutated lectins at least a part of which carbohydrates-recognizing regions are genetically engineered by random mutation can consist a lectin library as well.
Specifically, the terms "certain sugar chains" encompass the cases where sugar chains are defined at least partially, or sugar chains specified by a process of synthesizing/engineering the carbohydrates without knowing the exact structure. Further, the diversity (or an abnormality) in glycosylation of glycoproteins may result different sugar chains structure and this may also be an example of the subjects having "certain sugar chains" and glycoprotein itself can be used to select specific lectins from a lectin library. Further, cell surface sugar chains are characteristic and sugar chains are different from cells to cells, and thus "certain sugar chains" may be read as "certain cells" and "certain cells" can be used to select specific lectins from a lectin library. Further, if a cell has different cell surface sugar chains depending on the environment at the time of glycosylation, the cell cycle(for example a stage before differentiation or a differentiated state) may be identified. This is because glycosylation patterns depends on the cell cycle, environment and cell surface carbohydrates depends on diseases, aging etc.

The phrase "lectins having specific binding properties" is used because not all the mutated lectins have the same binding specificities, in terms of the binding preference to specific carbohydrates. When a parental lectin has a specific binding specificity to a specific carbohydrate structure, there is a high possibility that the mutated lectins generated from the gene mutation of its carbohydrates-binding site have a similar carbohydrate binding specificities pattern. However, mutated lectins have different binding preference each other and also different from that of the parental lectin. In this way, a plurality of lectins would be obtained, each showing different preference and capability to bind to a certain sugar chain.

The phrase "at least a part of which carbohydrates-recognizing regions" is given because lectins recognize carbohydrates by cross-linking to its carbohydrate-binding site, a certain region in its whole structure. The terms "at least a part" is given because gene mutation corresponding to the whole structure of the lectin is not always necessary.

The terms "genetically engineered" is given because chemical modification of an intact lectin is simply difficult and inefficient. Therefore; in the method, genes encoding "carbohydrate recognition regions" are genetically mutated and is transfected into a suitable host such as *Escherichia coli,* to produce a mutated lectin. The term "random mutation" means random genetic mutation, and also predetermined gene mutations to produce random lectins. The mutation used herein means the gene mutation by swapping nucleic acid base without changing the length of the gene or by making the length of the gene longer or shorter. This also applies to the mutated lectins produced from the mutations.

A lectin collection containing a plurality of lectins obtained in this manner may be called as a lectin library. A plurality of lectins is contained therein because a single lectin is not always sufficient for identifying various sugar chains.

Site-directed mutagenesis is performed because only the carobohydrate-binding site in the lectin molecule engages in the recognition of carbohydrates. By modifying such a specific site, a preferable lectin library would effectively be obtained. Of course, there may be the case where the effect of the lectin library can be evaluated from some experimental results, and thus it is not always necessary for the mutated site to be limited to such a specific site.

The method of identifying sugar chains (or a cell having a certain sugar chains) by a lectin library involves examining the binding of specific sugar chains to the lectins consisted the lectin library as described above, and then using the same lectin library to classify the carbohydrates to be identified, by evaluation the binding to the lectin library with two ranks, i.e. the presence of a binding ability (showing bonding) or the absence of a binding ability (showing no bonding) or with three ranks, i.e. the two ranks plus an intermediate rank or by multi-stage evaluation (or analog evaluation) with increased ranks, thereby distinguishing the type of the sugar chains in terms of the ability to bind to a plurality of lectins . Further, the method may also involve the identification/analysis of the binding ability by analyzing its binding pattern.

There may be various methods of judging the binding ability, and for example, the binding ability may be judged by comparison with a standard sample previously prepared.

As used herein, the "cells or pseudo-cells having predetermined sugar chains" have sugar chains, or mimic of the cell surface sugar chains. Accordingly, when a lectin sub-library is prepared for cell having carbohydrate A, carbohydrate A or mimic-A can usually be used to get an appropriate sub-library. The terms "analyzing sugar chain" may include identification of cells having sugar chain on the surface, diagnosis of sugar chain-modifidied serum glycoproteins (glycoform), diagnosis of diseases, and other analysis, and the "method of analyzing sugar chains" includes methods for conducting the above. The tool for diagnosing diseases by using a lectin sub-library for analyzing sugar chain, comprising a series of lectins prepared by introducing modifications at a predetermined site on the lectin, or plural types of lectins selected by panning with cells or pseudo-cells having predetermined sugar chains refers to a tool (diagnostic reagent, diagnostic apparatus, etc.) to judge the state of a disease (or health) when sugar chains on cell surface or glycoproteins are different in the two states, and a tool to examine the changes of the sugar chains through the lectin-binding specificities corresponding to the changes. It is not always necessary to determine the exact structure (or state) of the sugar chain.

The phrase "lectin-immobilizing regions are arranged in a suitable order for identifying/analyzing sugar chains, and the region contains a lectin selected for identifying/analyzing sugar chains" has a broad concept encompassing "wells or spots containing lectins selected for identifying cells, are arranged in a suitable and appropriate order for the cell identification", and the phrase "wells or spots containing lectins selected for identifying cells are arranged in a suitable and appropriate order for the cell identification" involves arranging wells or spots containing the selected lectins in predetermined positions so as to make their visual pattern easily recognizable for the identification, and if a standard pattern has been established, the phrase involves arranging the wells or spots in positions corresponding to those in the standard pattern. As used herein, "display" may be attained by either direct or indirect display. The chip can be used to provide a visual pattern obtained by sugar chain structure information, and on the basis of this information, the cells can be identified. To identify cells by enormous pattern information, visual judgment by the naked eye or any other methods is often insufficient, and thus the cells may be identified by cluster analysis with a computer. In the cluster analysis, a computer may be used to examine which part indicates a significant difference between subjects to be compared (for example, a standard and an object of examination or a control and an object of comparison), and general cluster analysis software can also be used.

In the phrase "solid carrier for analyzing/comparing sugar chains wherein different types of lectins are immobilized in a predetermined order", the terms "immobilized in a predetermined order" means that the position at which different types of lectin is immobilized, may be arranged depending on intention of using sugar chain information for cell analysis or disease diagnosis. This means, the order to immobilize lectins may be changed depending on the cell types and diseases. Similarly, in the "chip wherein wells containing lectins selected for identifying cells are arranged in a suitable and appropriate order for the cell identification", the order may be suitably varied depending on the intention of using sugar chain information for cell analysis or disease diagnosis. The order may preferably be optimized so as to be adapted to the intended cells or diseases. The solid carrier may be in a form of chip or a plate, or any other form of solid materials that can provide lectin-immobilizing regions. That is, the "solid carrier for analyzing/comapring sugar chains" includes "a test plate for analyzing sugar chains". The "test plate for analyzing sugar chains" includes a test paper using a paper such as a filter paper, or a glass plate or a substrate made of other material having predetermined lectins immobilized thereon. Other material for the substrate may include synthetic resin (including plastics), metals (including platinum, silver, copper, gold, silicon, etc.), mica and mixtures thereof. The "test plate for analyzing sugar chains" can also be used as a diagnostic reagent.

### Brief Description of Drawings

Fig. 1 shows the introduction of mutation in MAH carbohydrate recognition domain.
Fig. 2 is a photograph showing expression of recombinant MAH mutant lectins in transformed *Escherichia coli* induced by isopropyl thiogalactoside.
Fig. 3 is a list showing the amino acid sequences of the carbohydrate recognition domains of 16 mutant MAH clones.
Fig. 4 shows the hemagglutination activity of 16 mutant lectins (mutant MAH lectins) on erythrocytes from different types of animals.
Fig. 5 shows a mutation site in the carbohydrate binding site of MAH.
Fig. 6 is a graph showing the ability of wild-type MAH-expressing phage to bind to anti-MAH antibody.
Fig. 7 is a graph showing the ability of wild-type MAH-expressing phage to bind to human glycophorin.
Fig. 8 is a graph showing the sugar chain specificity of human erythrocyte-specific clones obtained by panning.
Fig. 9 shows the results of binding activity of the clones (1 to 18) to Caco-2 cells measured by a conventional cell-ELISA method.
Fig. 10 shows the results of binding activity of the clones (19 to 35) to Caco-2 cells measured by a conventional cell-ELISA method.
Fig. 11 is a graph showing binding patterns of differentiated or undifferentiated Caco-2 cells to the clones in a lectin library.
Fig. 12 are graphs showing binding patterns of various cells to commercially available lectins.
Fig. 13 illustrates one example of lectin chips and a method of using the same (for use in IgA discrimination).
Fig. 14 illustrates one example of lectin chips and a method of using the same (for use in profiling of differentiation of osteoblast).

### Detailed Explanation of the Invention

Hereinafter, the present invention is explained in more detail by reference to working examples.

At least a part of a region considered to correspond to the carbohydrates-binding site of *Maackia amurensis hemagglutinin* (MAH), which is a leguminous lectin specific for sialic acid-containing carbohydrates, is mutated at random by genetic engineering techniques, and from the resulting randomly mutated lectins, an artificial library was prepared capable of identifying the sugar chains variations in different type of sugar chains. Further, by using the resulting artificial lectin library, it was attempted to identify different type of cells based on the binding pattern to the lectins in the library. Then, from the artificial lectin library, a screening system established for selecting the lectins specific for biologically important sugar chains.

It is known that a vast variety of complex carbohydrates having slightly different structures, exist among mammalian cells. Formation of lectins capable of distinguishing such various structures is very useful.

Such lectins can be obtained from *Maackia amurensis hemagglutinin* (MAH) by genetic engineering techniques.

MAH can recognize a carbohydrates whose hydrocarbon sequence comprises a sialic acid residue, e.g. Neu5Acα2-3Galβ1-3(Neu5Acα2-6)GalNAc (4). Another isolectin engineering *Maackia amurensis leukoagglutinin* (MAL) can specifically recognize a sequence Neu5Acαx2-3Galβ1-4GlcNAc (5), and both lectins are unique among other lectins including leguminous lectins.

MAH is a dimer of subunits having a relative molecular mass of 29,000.

A nucleotide sequence of cDNA encoding MAH and an amino acid sequence deduced therefrom show that MAH consists of 287 amino acids and has a single peptide consisting of 30 amino acids.

The putative carbohydrate-recognizing domain of MAH was identified by comparing its amino acid sequence with amino acid sequences of other leguminous lectins (7) and by a study of genetic mutation on this domain defined by amino acids endowing MAH with binding characteristics.

Further, those observations were confirmed from a computer model of a three-dimensional structure of MAH containing Neu5Acα2-3Ga1β1-3 (Neu5Acα2-6) GalNAc (8).

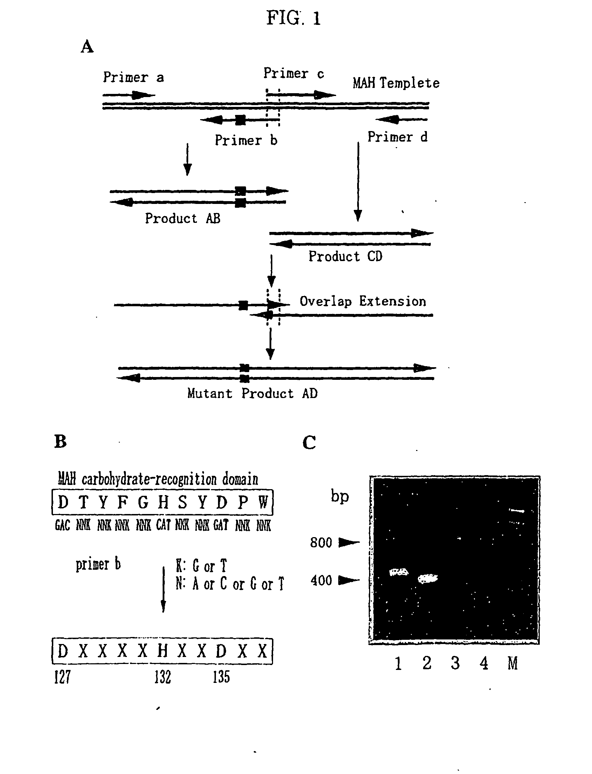

Figs. 1A to C are drawings showing the introduction of a mutation in MAH carbohydrate-recognition domain. A is an illustration showing a partial overlap extension method for introducing a mutation. 1B is a drawing showing the carbohydrates-recognition domain of MAH, where mutation is introduced at X sites. 1C is a photograph of agarose gel electrophoresis showing identification of a DNA of mutant MAH. In 1C, lane 1 is a product AB (up to 400 bp), lane 2 is a product CD (up to 400 bp), lane 3 is a product AD (up to 800 pb), lane 4 is a DNA of wild-type MAH (up to 800 pb) , M is DNA marker (100 bp DNA Ladder, Life Technologies, Inc., MD, USA).

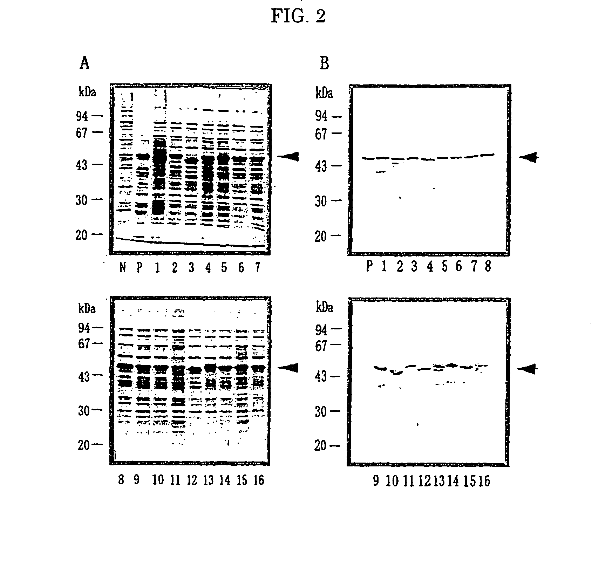

Fig. 2 is a photograph showing expression of recombinant MAH mutant lectins in transformed *Escherichia coli* induced by isopropyl thiogalactoside. 2A is a photograph showing the result of polyacrylamide gel electrophoresis analysis of recombinant mutant MAH lectins. 2B is a photograph showing the result of Western blotting analysis of recombinant mutant MAH lectins with an anti-MAH polyclonal antibody. In Fig. 2, lane N represents *E. coli* carrying pGEX-2T plasmid, lane P represents *E. coli* carrying pGEX-2T/wild-type MAH plasmid, and lanes 1 to 16 represent *E. coli* carrying pGEX-2T/mutant MAH plasmids (corresponding to clones 1 to 16, respectively). Further, their relative molecular masses are shown in the left, and the arrow indicates recombinant MAH mutant lectins.

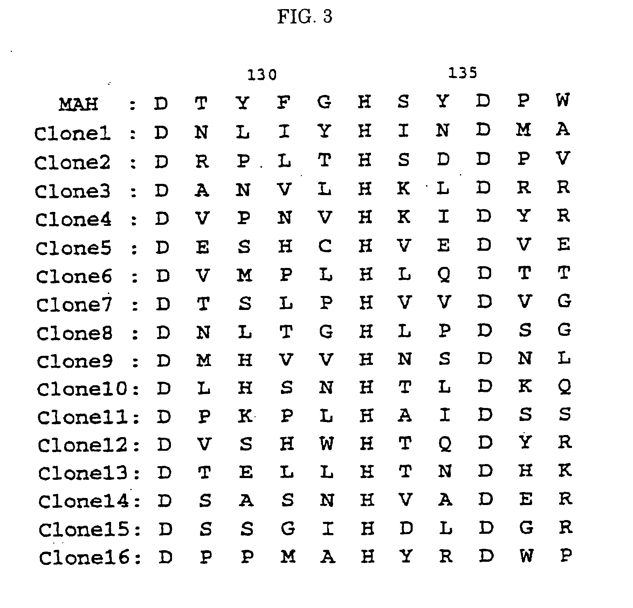

Fig. 3 is a list showing the result of deduced amino sequences of sugar-recognition domains in 16 mutant MAH clones.

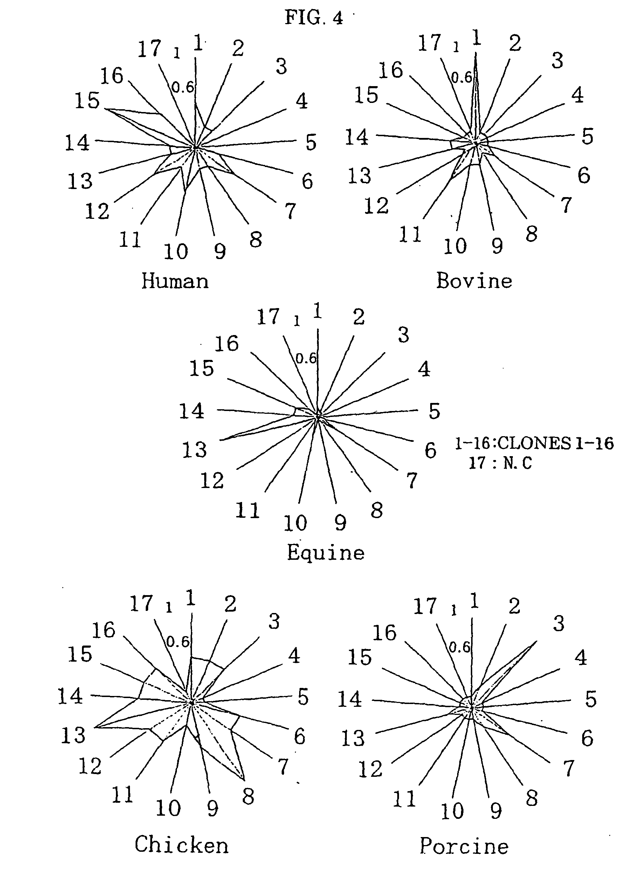

Fig. 4 are graphs showing the result of the hemagglutination activities of 16 mutant lectins on erythrocytes from different kinds of animals. The activity of each mutant lectin is shown in terms of minimum hemagglutination concentration relative to maximum hemagglutination concentration assumed to be 1. Nos. 1 to 16 refer to clones 1 to 16, and No. 17 refers to a negative control (GST).

### <Example 1>

### [Preparation of an artificial lectin library]

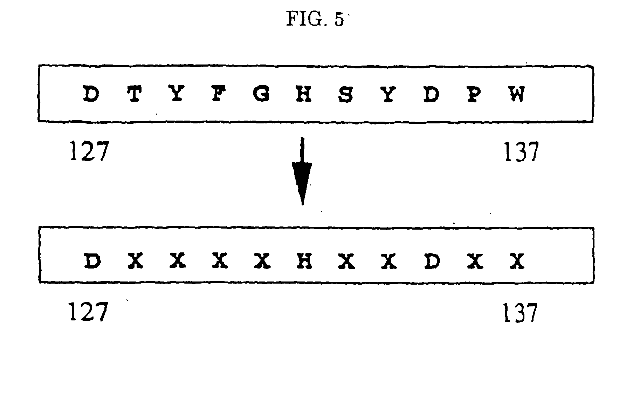

On the basis of information obtained from the amino acid sequences of various leguminous lectins and carbohydrate-binding sites thereof, a nucleotide sequence encoding the carbohydrate-binding site at the 127- to 137-positions in 285 amino acids of MAH lectin was randomized by overlap extension PCR using synthetic oligonucleotides primers. However, aspartic acid 135 considered to be essential for interaction with sialic acid and aspartic acid 127 and histidine 132 considered to be essential for coordination with a metal ion were fixed (Fig. 5). On determining the DNA sequence by the dye terminator method, it was confirmed that the obtained mutant MAHs, excepting the initially fixed 3 amino acids, have different amino acid sequences with each other, indicating that the randomization of MAH lectin has been attained.

### [Discrimination and identification of cells by the lectin library]

The prepared lectin library was integrated in a vector pGEX and expressed as GST fusion protein in *Escherichia coli.* Fifty clones were selected at random, and from these clones, 16 clones considered to express the protein were obtained. Western blotting of the proteins with an anti-MAH polyclonal antibody indicated that all the clones remained reactive. After the determination of the DNA sequences of these clones, it was confirmed that the amino acid sequences of the sugar-binding sites in all clones, excepting the fixed amino acids, had been changed.

By utilizing a difference in sugar -binding specificity among the resulting 16 clones, it was attempted to distinguish and identify erythrocytes of different kinds of animals, on which sugar chains comprising a sialic acid but having different structure is considered to exist. Fig. 4 shows the result of the experiments where the agglutination titer of each lectin of the 16 clones is compared. In the graphs, the data were standardized by taking the maximum agglutination titer shown by the most reactive lectin for the respective erythrocytes as "1" (full-scale). The respective erythrocytes were found to show inherent characteristics in terms of the relative agglutination titer against the 16 mutant lectins.

This experiment suggests that the difference in the type of cell can be distinguished by referring to the binding patterns with respect to a plurality of lectins.

### [Preparation of a phage library]

The artificial lectin library would be further developed by selecting lectins specific for a biologically useful carbohydrate chain. For this selection, a system capable of expression cloning and dealing with a large number of libraries should be used. In this context, a phage display system was introduced. It was attempted to use an expression vector λfoo and then a T7 phage display system. However, the growth of lectin-expressing phage was delayed as compared with the counterpart phage not carrying the gene, and it was found difficult to concentrate the desired phage by panning. (However, this does not necessarily mean that the expression vector λfoo or the T7 phage display system is excluded from the present invention.)

Incidentally, panning refers to a technique of recovering phage by utilizing the ability of a protein expressed on the phage to bind to the binding partner (for example, the binding ability between antigen and antibody, lectin and carbohydrate, receptor and ligand, etc.) For example, a substance that binds to the protein expressed on phage is immobilized on a plate, then a phage solution is added to the plate thereby allowing the expressed proteins, phage and other materials to bind to the said substance, then the phage solution is discharged, and the expressed proteins, phage and other materials thus binding to the substance are recovered. The recovered phage can be used to infect with *Escherichia coli* to proliferate and then subjected again to (the above-described) panning to recover highly binding phage.

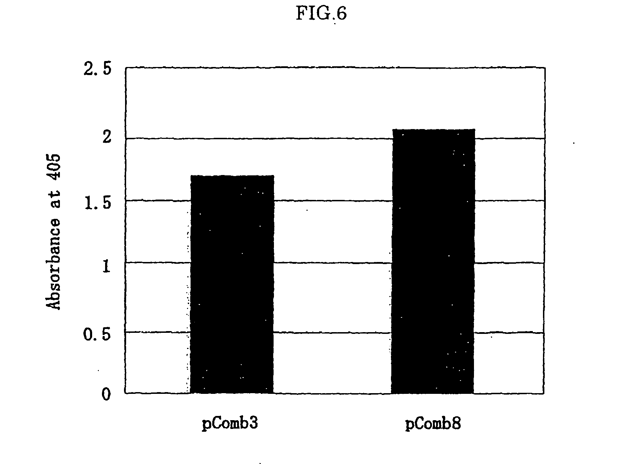

Then, it was attempted to use phage imids pComb3 and pComb8 . It was first examined whether a lectin expressed on the surface of both the phages had an activity or not by expressing wild-type MAH. When the phages considered to express wild-type MAH were examined for their reactivity with an anti-MAH antibody, both phages were bound specifically to the anti-MAH antibody. pComb8 expressing a plurality of MAH proteins showed a stronger binding ability than pComb3 expressing a single MAH protein (Fig. 6).

It was examined whether the wild-type MAH expressed on the phages was maintaining the carbohydrate-binding activity by measuring the ability to agglutinate erythrocytes. Both the phages considered to express wild-type MAH agglutinated untreated erythrocytes, but did not agglutinate erythrocytes treated with siliadase. The minimum titer of both the phages necessary for hemagglutination was about 1 × 10¹⁰ cfu. Thus, it was revealed that the activity of MAH can be maintained on the phage, and therefore MAH having a carbohydrate binding activity can be expressed on the pComb phage.

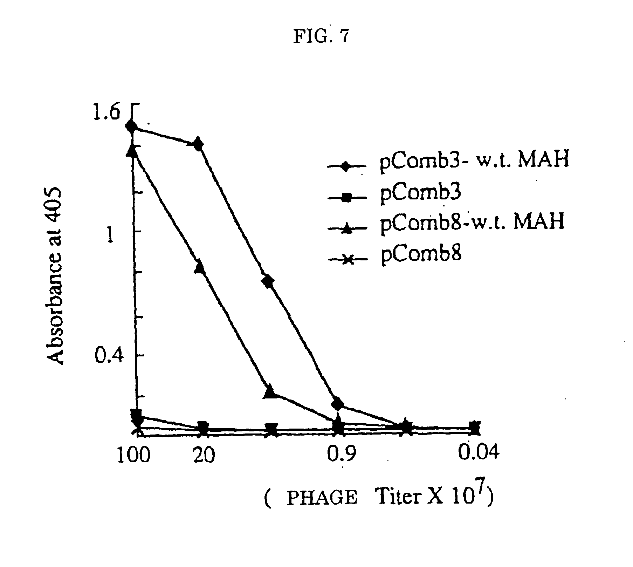

The activities of both phages were further examined in more detail in terms of the ability to bind to human glycophorin (sialic acid-containing glycoprotein). It was unexpectedly found that pComb3 expressing a single MAH protein per phage had a higher activity (Fig. 7). The minimum titer of pComb3 phage detectable by ELISA in terms of the ability to bind to human glycophorin was about 1 x 10⁷ cfu.

### [Selection of specific lectins from a phage library]

On the basis of the above results, the pComb3 phageimid was used to prepare a lectin library having mutated primary structures. For preparation of the library in a feasible range, 3 amino acids that were considered to be essential for interaction with sialic acid and for coordination with a metal ion, were fixed. Other two amino acids that are highly conserved among other leguminous lectins, were also fixed. The remaining 6 amino acids were mutated at random in the amino acid sequence of the MAH sugar-binding site. As a result, a library of about 1 × 10⁸ lectins considered to cover the theoretically estimated number of lectins of 6.25 × 10⁷ could be prepared.

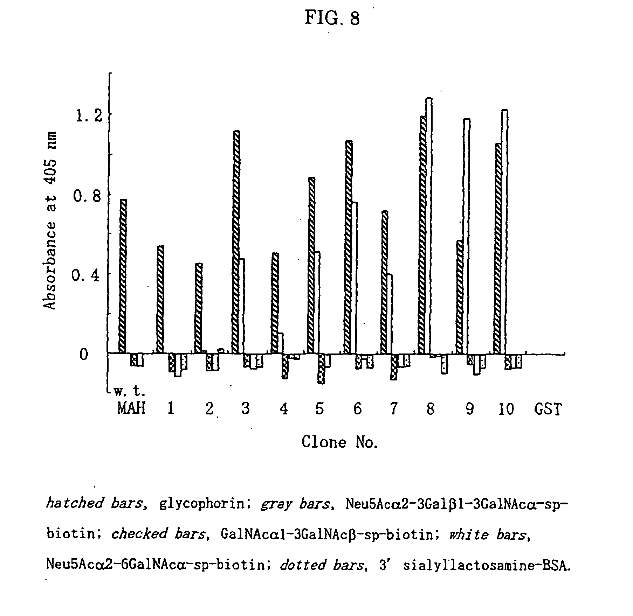

For establishing a panning system for the lectin library of pComb3 phage, a panning with human erythrocytes that strongly bind to the wild-type MAH was performed. The recovery in the third panning was about 10 times as high as that in the first panning, thus revealing that the phage having an ability to bind to human erythrocytes was concentrated. From the phages obtained after the third panning, several clones that specifically agglutinate human erythrocytes but shows different carbohydrates specificity were obtained (Fig. 8). In this way, a screening system could be established for selecting lectins specific for biologically important carbohydrates from a library of lectins having mutated primary structures.

### [Mutagenesis of the sugar chain recognition site of MAH]

The carbohydrates recognition site of MAH was randomized by site-directed mutagenesis (9, 10) utilizing overlap extension procedure. In the overlap extension, the following 4 primers were used.

**Table 1. Four primers**

| | |
|---|---|
| Primer a : | 5'-ccccggatccacatcagatgagctttct-3' (bp 89-105) |
| Primer b : | 5'-atgtcgataatttggatctnnmnnatcmnnmnnatgmnnmn nmnnmnngtcaaactctacagc-3' (bp457-519) |
| | (N represents A, T, G or C, and M represents A or C) |
| Primer c : | 5'-gatccaaattatcgacatatcggaattgat-3' (bp 502-531) |
| Primer d : | 5'-cccgaattcagatcatgcagtgtaacg-3' (bp 847-531) |

MAH cDNA fragment was PCR amplified by using primer pairs of a and b or c and d with AmpliTaq Gold DNA polymerase (Perkin Elmer) . Standard PCR conditions were used. Specifically, the reaction mixture was incubated at 95°C for 9 minutes and at 94°C for 1 minute and then subjected to 30 cycles each consisting of incubation at 94°C for 1 minute, at 54°C for 1 minute and at 72°C for 1 minute followed by one cycle of incubation at 72°C for 10 minutes, whereby all amplified fragments were extended certainly to the 3' end. The 3'-end of the PCR product was polished with a pfu DNA polymerase (Stratagene La Jolla, California). The PCR product was purified and quantified by preparative agarose gel electrophoresis. Equimolar amounts of the two PCR fragments were bound to each other by 7 cycles each consisting of incubation at 94°C for 1 minute and at 63°C for 4 minutes. The product was used as template DNA in second reaction using primers a and d, whereby a full-length mutant MAH cDNA was prepared.

### [Expression and purification of GST fusion protein]

The PCR product containing the full-length mutant MAH cDNA was digested with BamHI and EcoRI and then ligated to pGEX-2T previously digested with BamHI and EcoRI. *E. coli* BL21 strain containing the expression plasmid was cultured in a 2× yeast-trypton medium supplemented with 50 µg/ml ampicillin, and grown at 37°C until the absorbance at 660 nm reached 0.8. Expression of GST fusion protein was induced by adding isopropyl β-D-thiogalactoside (final concentration: 1 mM) and then the cells were incubated at 37°C for 3 to 4 hours. Apellet of the microbial cells was suspended in Tris buffered physiological saline (TBS), and sonicated. The insoluble fraction was washed twice with TBS and dissolved in 8 M urea in TBS . Thus solubilized inclusion body was refolded by 50-fold dilution with TBS/1 mM MnCl₂/l mM CaCl₂. The supernatant was applied onto a glutathione-Sepharose 4B column (Amersham Pharmacia Biotech UK Ltd., England), and the GST fusion protein was purified according to the manufacturer's instructions.

### [Detection of GST protein by Western blotting]

The microbial lysate (0.5 µg protein/ml) was separated by sodium dodecylsulfate-polyacrylamide gel electrophoresis, transferred onto a nitrocellulose membrane, screened by polyclonal anti-MAH antibody and then incubated with an alkali phosphatase-bound anti-rabbit IgG antibody (ZYMED, San Francisco, California). According to manufacture's instructions, the bound antibody was visualized with an alkali phosphatase substrate kit SK-5200 (Vector Laboratories, Beringam [phonetic], California).

### [Determination of DNA sequences of recombinant MAH mutants]

DNA sequences of 16 clones selected at random were determined by using a sense primer 5' -ttcttgcaccacctgattctc-3' and an antisense primer 5'-ccgccgttaatccaatcccat-3' with ABI PRISM^{™} Dye Terminator Cycle Sequencing Ready Reaction Kit (Applied Biosystems, California, US).

### [Preparation of erythrocytes]

Human venous blood of the O type was taken from a blood donor by using a previously heparin-treated hypodermic syringe. The heparin-treated blood was transferred into a glass cylinder and left until the erythrocytes were precipitated by gravity. After leukocyte-rich serum and buffy coat were removed, the erythrocyte layer was washed 3 times by centrifugation with phosphate buffered saline (140 mM NaCl, 2.7 mM KCl, 10 mM Na₂HPO₄, 1.8 mM KHPO₄ (pH 7.3)), and the outermost layer was removed carefully each time of washing after centrifugation. It was confirmed that the erythrocytes obtained in this manner did not contain leukocytes or cell debris. Bovine, equine, porcine and chicken erythrocytes were prepared in the same manner.

### [Hemagglutination assay]

A recombinant protein purified from each of the 16 mutant lectins was diluted twice and put to a U-bottomed plate (Nunc, NY, USA), and to each dilution (0.05 ml) was added an equal volume of 2% erythrocyte suspension. The mixture was left for 1 hour at room temperature and then examined for hemagglutination.

The following gives more specific disclosure.

### [Mutagenesis of the sugar chain recognition site of MAH]

The overlap extension method (9, 10) was used to introduce a mutation into the sugar chain recognition site of MAH. The scheme (strategy) of this method is shown in Fig. 1.

Using MAH cDNA cloned in pGEX-2T as a template for PCR amplification, two different DNA fragments (AB and CD) were formed. The fragment AB was amplified with primers a and b. Because Asp 135, Asp 127 and His 132 were considered to participate in binding of MAH to sialic acid, primer b was designed such that while Asp 127, His 132 and Asp 135 resides were stored, the sequence of the sugar chain recognition site of MAH was mutated (Fig. 1 B).

The fragment CD was amplified with primers c and d. The 5' end of the primer c had a 12-bp sequence found in the 3' end of the fragment AB. The fragments AB and CD can be ligated to each other via this common 12-bp sequence by the overlap extension method to form an AD product harboring the mutant MAH cDNA. The length (up to 800 bp) of the mutant MAH cDNA was confirmed by agarose gel electrophoresis (Fig. 1C).

### [Expression of MAH mutant lectins and elucidation of characteristics thereof]

The mutant MAH cDNA was inserted into a BamHI-EcoRI site in E. coli expression vector pGEX-2T containing a glutathione-S-transferase (GST) gene upstream of the insertion site. GST/mutant lectin fusion proteins were expressed by using *Escherichia coli* BL21 as a host strain. The ability of 50 randomly selected clones to express the recombinant mutant lectins was examined by polyacrylamide gel electrophoresis. GST/mutant lectin fusion proteins from 16 clones were electrophoresed to a position corresponding to an apparent molecular weight of 55, 600, which is predicted with an molecular weight of the GST/mutant lectin fusion product (Fig. 1A).

Plasmid DNAs from the 16 clones were sequenced by a dye deoxy chain terminator method (11). Fig. 3 shows the elucidated amino acid sequences of the sugar chain recognition sites of these clones. As expected, the sugar chain recognition sites of the 16 clones were different in amino acid sequence excepting Asp 127, His 132 and Asp 135 residues. Regardless of this difference, all the 16 mutant lectins were detected by Western blotting with an antibody specific for MAH (Fig. 1B).

The 16 mutant lectin fusion proteins were purified by affinity chromatography on a glutathione-Sepharose column. The concentration was determined by a BCA protein assay kit with bovine serum albumin as the standard. Profiles of the purified fusion proteins after polyacrylamide gel electrophoresis in the presence of SDS suggest that all fusion proteins have a molecular weight of 55,600. These proteins were used in hemagglutination assays.

### [Hemagglutination activity of the mutant lectins]

Hemagglutination assays using human, bovine, equine, porcine and chicken erythrocytes revealed that there was a significant difference in the sugar chain specificity among the 16 mutant lectins and the wild-type MAH (Fig. 1) . Sugar chains on the surfaces of these erythrocytes are different in the structure, amount and expression pattern as is evidently shown by comparing the respective recognition patterns by the mutant lectins (Fig. 4). Each type of erythrocyte can be easily identified by using these lectins.

In a radial graph in Fig. 4 similar to a form of bicycle spokes, the 16 different lectins and blank are expressed as straight lines of the same length, and these straight lines are arranged radially from one point at equal angles through 360°. In Fig. 4, one radial graph is prepared for erythrocytes from one type of animal, and this graph shows the discrimination (or characteristics) of the erythrocytes by the respective lectins in a visual manner. That is, the maximum agglutination titer observed is expressed in full scale "1" and plotted in the outermost side in this graph. The agglutination titers of the other lectins are plotted in a similar manner, respectively, and adjacent plots are linked via a straight line. By expressing plots in this manner, the positions of the plots is more easily recognizable than by simple plots, and the resulting graph in a web form permits discrimination (or characteristics) of the erythrocytes by the respective lectins to be easily and visually recognized. The 16 lectins can be arranged so that the similar ones are placed as adjacent as possible, contrastive ones are placed apart at 180°, and so on. It is preferable that the arrangement of the plot of lectins is suitably coordinated depending on the sugar chain to be analyzed. The graph in Fig. 4 shows all the 16 lectins, but the graph may be modified to show 15 or less lectins, wherein some lectins not necessary for discrimination are omitted, or it may of course include 17 or more lectins adding thereto some additional lectins. Further selection of such lectins can be carried out, for example, from the viewpoint of their ability to bind to intended sugar chains. Referring to Fig. 4, clone 15 can be selected for distinguishing bovine erythrocytes from human erythrocytes. This is because clone 15 has an ability to bind strongly to human erythrocytes but is poor in an ability to bind to bovine erythrocytes and the relative difference therebetween is great. Further, clone 13 may also be selected. This is because clone 13 has the same ability to bind to human erythrocytes and bovine erythrocytes and the relative difference therebetween is small. By selecting lectins showing great and small relative differences respectively for intended erythrocytes, unknown samples of erythrocytes could be judged easily and reliably.

The life cycle of cells in multi-cellular living things is regulated by stimuli from extracellular environments, and such stimuli are mediated at least partially via sugar chain and its recognizing molecules. It has been considered that, as in the case of receptors specific for antigens in an adapted immune system, cell surface carbohydrates have a wider variety. However, there is a limit to the variation of natural lectins capable of recognizing such tremendous variety of carbohydrates/oligosaccharides.

A method is provided that enables the preparation of a number of novel lectins (novel lectin library) having various specificities. The 16 mutant MAH lectins were produced. Although their characteristics in terms of carbohydrate binding specificity has not fully been revealed, it is noteworthy that the mutants can agglutinate human and animal erythrocytes with highly specific patterns. The specificity of lectins to distinguish different animal erythrocytes from one another has not been achieved except by using specific antibody thereagainst. The present invention is to prove that a panel of genetically engineered lectins having various specificities is useful for distinguishing the type of different cells.

In the last several years, DNA arrays techniques were established as a new technology for distinguishing the type of difference cells thorough profiling of gene expression (15). However, the phenotype of cells cannot fully be determined only on the basis of gene expression. Posttranslational modification by adding sugar chains and control of protein localization are considered to have essential roles in determining the actual stage in the differentiation of certain type of cells. Accordingly, different cells should be distinguished by describing the state of glucan on the surfaces of the cells. Use of a panel of lectins having various specificities is the most convenient and effective means for achievement thereof. Development of a novel lectin library can significantly expand the applicability of lectins to ultimately make the identification of cells accurate and reliable. Although the expression of carbohydrates are very low, and their structure is complicated, the lectin library and/or lectin sub-library according to the present invention can be used to provide a convenient discrimination (or identification) method that can also be used in clinical fields.

### <Example 2>

### [Cell discrimination and identification targeted at Caco-2 cells]

A mutant MAH lectin library consisting of loop D mutated MAH lectins was subjected to panning with differentiated Caco-2 cells as standard cells, and clones used as probes for cell discrimination and identification of the target Caco-2 cells were recovered. After the second panning, the concentration of the binding clones was considered insufficient, and thus in the third panning, the cells were fixed with glutaraldehyde, and the reaction time in the panning procedure was changed into o/n.

### [Determination of DNA sequences of recovered clones]

After the third panning, 64 clones were selected at random and analyzed for their sequence. The 64 clones consist of 35 clones having the intended mutations, 7 clones having a stop codon, 17 clones undergoing frame-shift, and 5 clones that could not be analyzed. Among 35 clones having the intended mutations, there were no clones having the same sequence as that of the wild type and he clones have no common sequence at the mutated site (Table 2).

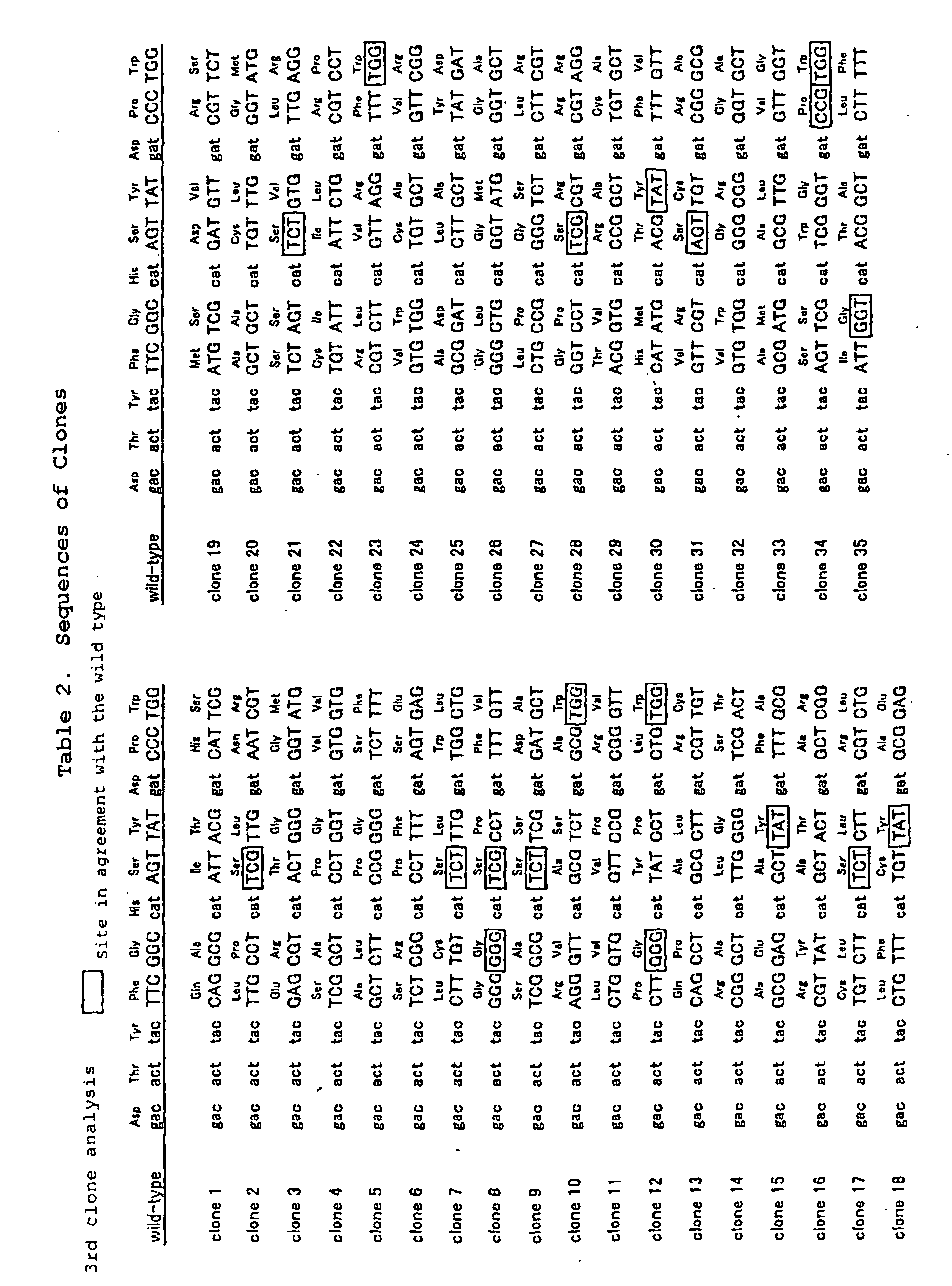

A large number of clones different from the wild type has been obtained, these 35 clones can be used as probes for discrimination and identification of cells. On the other hand, since no common sequence was detected among these clones, and clones having a stop codon or undergoing frame-shift were recovered, the concentrating efficiency in the panning procedure is considered as not so high in this experiment.

### [Measurement of the activity of the recovered-clones]

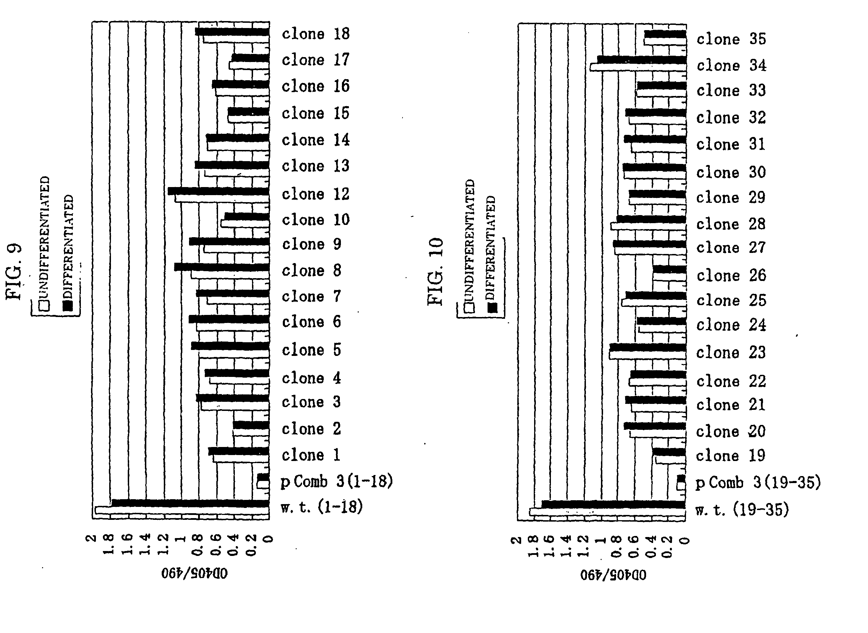

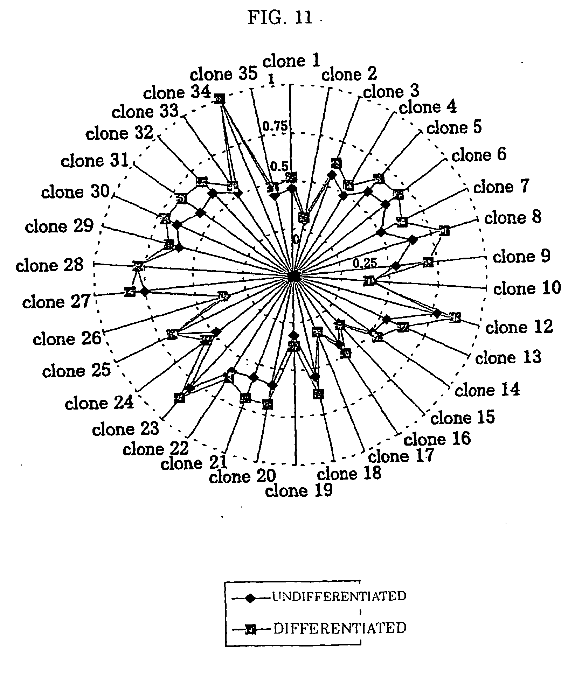

Using a lysate of each clone, the activity thereof to bind to Caco-2 cells was measured by a conventional cell-ELISA method (Figs. 9 and 10). All of the 35 clones had an activity to bind to both differentiated and undifferentiated Caco-2 cells. When binding patterns of the respective clones were plotted with respect to the differentiated and undifferentiated Caco-2 cells, a binding pattern appeared to be unique to Caco-2 cells was obtained and there was a slight difference in the pattern between the differentiated and undifferentiated cells (Fig. 11). These results indicate that a difference in the pattern between the differentiated and undifferentiated cells can be observed even in the same kind of cells. This type of graph in Fig. 11 is superior in judging a slight difference because the results of both the differentiated and undifferentiated cells can be collectively shown in one graph.

### <Example 3>

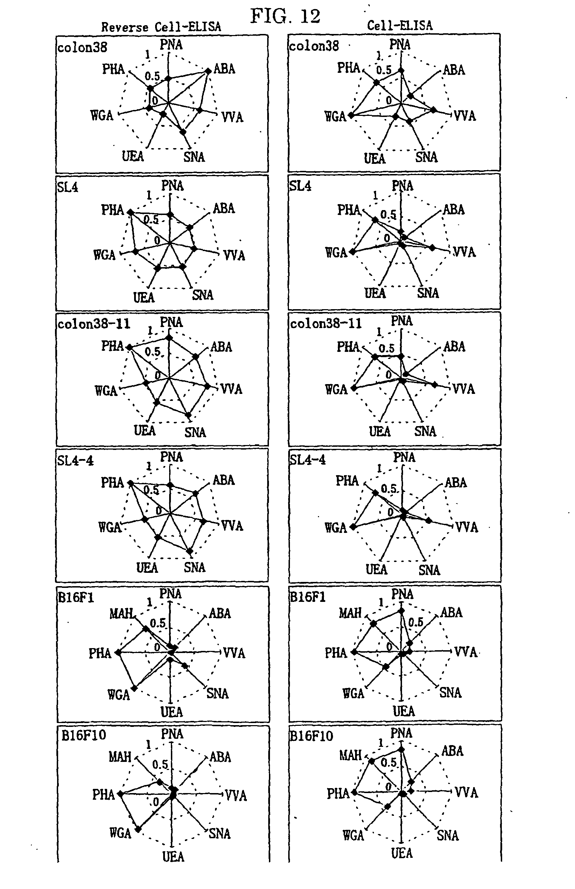

Profiling of various cells was conducted as well. The commercially available purified lectins lectins (PNA, ABA, VVA-B4, SNA, UEA-I, WGA, PHA-L4) were immobilized directly onto wells and sample cells were added to the wells to detect the binding thereof (reverse cell-ELISA method) (Fig. 12). As can be seen from the graphs in Fig. 12, a binding pattern unique to each kind of cell is obtained and expressed in a graph in a radial form similar to a form of bicycle spokes, whereby characteristics of the cell can be grasped. The manner of plotting the graph in Fig. 12 is the same as that in Figs. 4 and 11 described above.

### [Discrimination of IgA glycoforms]

An application of the lectin library to a method of distinguishing IgA glycoforms is described below.
1) A genetically mutated lectin library is prepared by mutating an amino acid sequence of the sugar chain recognition site of MAH lectin (Maackia amurensis hemagglutinin lectin).
2) Lectins having higher affinity for an IgA from a patient having IgA nephropathy are selected by the panning method, to prepare a lectin sub-library.
3) Lectins from the lectin subolibrary obtained in step 2) above, are immobilized on a microtiter plate. Optionally, lectins sufficiently reflecting a difference between the IgA from the patients having IgA nephropathy and IgA from healthy persons may be specifically selected from the lectin sub-library obtained in step 2) above, to prepare the plate.
4) Discrimination by a lectin library of IgA glycoforms: Binding patterns, on the lectin plate, of serum IgA from the patients having IgA nephropathy and serum IgA from healthy persons are compared and analyzed,
5) Investigation of assay conditions for serum analysis: Sugar chains are added enzymatically or chemically to IgA from healthy persons, to prepare artificial IgA. The artificial IgA is mixed with serum from healthy persons and subjected to pattern analysis by using the lectin plate. The influence of other serum proteins present in serum may be examined to optimize assay conditions.

IgA nephropathy is a disease in which IgA is precipitated in renal glomerulus, and the IgA molecule is different in the sugar chain structure on its hinge region between the IgAs from patients and healthy persons. The analysis of the sugar chain structure is conventionally carried out by purifying serum IgA, separating the hinge-containing peptide fragment by a treatment with trypsin, and measuring its mass by a mass spectrometer. The hinge region refers to a region between two H-chain constant domains constituting an immunoglobulin molecule, and it is assumed that in patients with IgA nephropathy, there is an abnormality in an O-linked sugar chain on the hinge region in IgA. Further, an O-linked sugar chain having, as a fundamental skeleton, a structure composed of N-acetyl galactosamine (GalNAc), galactose (Gal) and outermost sialic acid, is also referred to as a mucin type sugar chain. The 0-linked type sugar chain can significantly influence on such as intercellular interactions, cellular infiltration, adhesion etc.

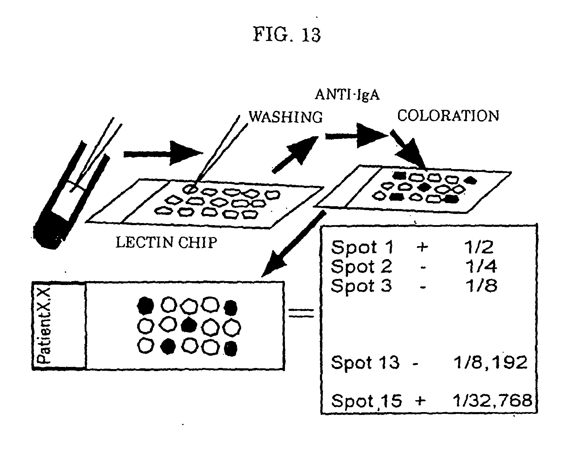

The detailed mechanism of the method is as follows: The O-linked type sugar chains can be attached onto serine and threonine residues at 5 distinctive sites in the amino acid sequence of the hinge region in IgA, where patients with IgA nephropathy have an abnormality in the O-linked sugar chain. There are 6 different patterns of the O-linked sugar chains, and thus 6⁵ (i.e. 7,776) different sugar chain structures can theoretically occur in relation to the attaching positions in the hinge region. It is difficult to examine every possibility by a complicated sugar chain analysis conducted conventionally. However, the pattern analysis would be feasible by observing ON/OFF signals toward lectins that recognize the 0-linked sugar chain. By examining ON/OFF toward one lectin, two sugar chain linkage modes can be determined, and with "n" lectins given, 2ⁿ patterns would be recognizable. Accordingly, the number of lectins necessary for analyzing 7, 776 sugar chain abnormalities in the hinge is theoretically 13. This theoretical number is elucidated based on an assumption that there are lectins capable of clearly reflecting the structural and positional information of the sugar chain. However, if information from several hundred lectins is available and the lectins can be blotted on a substrate, there is a high possibility that the structural and positional information of the sugar chain in the IgA hinge can be obtained. To obtain a useful lectin library, a group of lectins that clearly reflects the difference in between serum IgAs from patients and from healthy persons, can be identified by a cluster analysis to construct an effective lectin sub-library. Using the lectin library, a difference in glycoform between serum IgA from patients with IgA nephropathy and serum IgA from healthy persons is analyzed by the pattern information (Fig. 13). Fig. 13 shows that the profiling of IgAl, a glycoprotein containing a plurality of O-glucans, can easily be carried out. There is applicability not only to analysis of IgA from patients with severe diseases such as those in chronic renal insufficiency but also to early diagnosis of potential patients with a slight symptom not diagnosed as IgA nephropathy.

### [Method of distinguishing osteoblast subgroup]

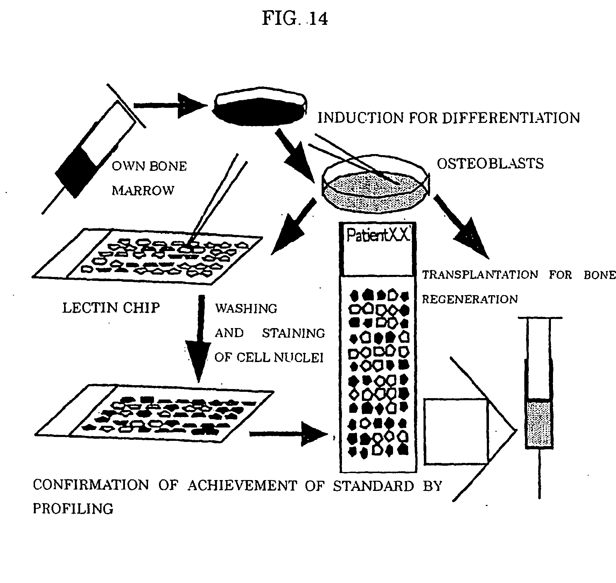

One example of the application of the lectin library to a method of distinguishing osteoblasts or to a method of detecting a subgroup different in the stage of differentiation is described below.
1) A genetically mutated lectin library is prepared by mutating an amino acid sequence of the sugar recognition site of MAH lectin (*Maackia amurensis hemagglutinin* lectin).
2) Lectins having high affinity for the prepared osteoblasts are selected from the lectin library by panning to prepare a lectin sub-library.
3) Lectins from the lectin sub-library obtained in the step 2) above, are immobilized on a microtiter plate. Optionally, lectins sufficiently reflecting the stages of differentiation are selected from the lectin sub-library obtained in the step 2) above, to prepare the lectin plate.
4) Introduction of the differentiation of osteoblasts and discrimination by the lectin library: Mesenchymal stem cells are cultured and separated. Specifically, the mesenchyme stem cells are cultured and then differentiated into osteoblasts, and the cells on the 5th day, 10th day, 15th day and 20th day after the initiation of differentiation are separated.
5) The cells separated at each stage in the differentiation process are analyzed with the lectin plate. The correlation between the sugar chain structure on the surface of the cell and the ability to form bone is examined. The ability to form bone is determined by measuring the activity of bone-type alkali phosphatase and simultaneously measuring the content of osteocalcin (preparation of a standard). That is, the separated cells in the form of a complex with a b-TCP block serving as an anchor for the osteoblasts are transplanted subcutaneously in the back of a rat, and 4 weeks and 8 weeks after transplantation, the cells are removed and examined for (i) osteocalcin content and (ii) bone-type alkali phosphatase activity.
6) Cells whose differentiation process is not identified/ unrevealed are analyzed with the lectin plate and compared with the standard pattern (see Fig. 14). Fig. 14 shows that profiling of differentiation of osteoblasts can be easily conducted.

As used herein, the mesenchymal stem cells are stem cells which among those differentiated into tissues or organs, occur in the bone marrow, and it is confirmed that the mesenchymal stem cells are differentiated into cells in bone, cartilage, fat, heart, nerves, liver etc., and have a potential similar to that of embryonic stem cells (ES cells) which can be differentiated into almost all tissues. The mesenchymal stem cells can be used as an anchor for culturing an artificial material based on calcium phosphate called b-TCP (b-tricalcium phosphate) excellent in affinity, absorptivity, and bone conductivity in the living body.

The lectin library was found to be useful for discrimination and identification of cells.

By analyzing cell surface sugar chain by this new method, identification of cells, which has solely been depended on gene expression only, or antibodies whose antigen recognition is limited, be easily carried out, and thus this method would be useful for development of cell transplantation and cell therapy. Further, this study succeeded in expressing an artificial lectin library on the surface of phageimid-type phage. Because the method of selecting lectins having different specificities from a mutant lectin library was established, it is expected that this system can be used for obtaining lectins having novel sugar chain specificity absent in existing lectins or monoclonal antibodies.

Further, the lectin library can be used to provide a tool for detection of IgA glycoforms or for discrimination/identification of an osteoblast subgroup. That is, the lectin library can provide not only a diagnostic reagent for easily and rapidly analyzing glycoforms of various serum proteins including IgA, but also a standard design tool for guaranteeing qualities of cells necessary for bringing regenerative medical treatment or cellular medical treatment into a practical stage. For example, these tools can be applied to early discovery of diseases, to accurate understanding of a morbid state and to therapeutic/prophylactic medicines, for example by analyzing glycoforms of immunoglobulins in rheumatism and autoimmune diseases, glycoforms of specific hormones in patients with cancers (that is, change in sugar chains of chorionic gonadotropin in ovarian cancer) and glycoforms of proteins (change in sugar chains of alpha-fetoprotein at a stage of from hepatitis to hepatoma).

Because an antibody is characterized by being produced depending on a difference in species, an antibody specific for fibroblasts is hardly produced. Therefore, fibroblasts derived from bone marrow do not have a clear so-called cell surface marker, and there is no method of directly assaying osteoblasts. Even at present time, bioassays with animals or indirect bioassays reflecting the activity of osteoblasts are employed. In such a field, a lectin chip using the lectin library would be feasible in the discrimination/identification of osteoblasts, particularly in the discrimination/identification of an osteoblast subgroup at different stages. By the same approach, it can duly be expected that the lectin library can be applied to the analysis of the dendritic cells derived from bone marrow and the vascular endothelial cells derived from bone marrow, and therefore can be used as a standard tool for securing qualities of cells used in regenerative medical treatment.

A network for remote diagnosis of cancers at present is based on pharmacological diagnosis, but it is examined to add a genetic information thereto. On finding the considerable involvement of the O-linked sugar chain in the intercellular interactions, cell infiltration, adhesion etc., it would be possible to achieve more detailed remote diagnosis of cancers.

## Claims

1. A method of analyzing sugar chains in a sample by using a lectin library, wherein the lectin library comprises at least one recombinant *Maackia amurensis* hemagglutinin lectin molecule of which the carbohydrate-binding region comprises an amino acid sequence selected from the group consisting of
Asp Thr Tyr Gln Ala His Ile Thr Asp His Ser,
Asp Thr Tyr Leu Pro His Ser Leu Asp Asn Arg,
Asp Thr Tyr Glu Arg His Thr Gly Asp Gly Met,
Asp Thr Tyr Ser Ala His Pro Gly Asp Val Val,
Asp Thr Tyr Ala Leu His Pro Gly Asp Ser Phe,
Asp Thr Tyr Ser Arg His Pro Phe Asp Ser Glu,
Asp Thr Tyr Leu Cys His Ser Leu Asp Trp Leu,
Asp Thr Tyr Gly Gly His Ser Pro Asp Phe Val,
Asp Thr Tyr Ser Ala His Ser Ser Asp Asp Ala, and
Asp Thr Tyr Arg Val His Ala Ser Asp Ala Trp
and wherein said sugar chain is selected from the group consisting of glycophorin, Neu5Acα2-3Galβ1-3GalNAcα-, GalNAcα1-3GalNAcβ-, Neu5Acα2-6GalNAcα- and 3'sialyllactosamine- residues.

2. The method according to claim 1, wherein the lectin molecules in said lectin library are selected by panning with cells or pseudo-cells having predetermined sugar chains.

3. The method according to claim 1 or 2 by which cells having sugar chains are discriminated and/or identified.

4. The method according to claim 3, wherein the cells are discriminated and/or identified by comparing the bindings of the cells to different lectin molecules after characterizing the binding pattern by cluster analysis.

5. The method according to any one of claims 1 to 4, wherein the lectin library is formed as an array of lectins on a solid substrate and the different lectin molecules in said library are immobilized to predefined regions of the substrate.

6. The method according to claim 5, wherein the predefined regions to which the different lectin molecules are immobilized are arranged in a suitable order for characterizing a cell bearing a cell surface sugar chain.

7. The method according to claim 5 or 6, wherein the binding pattern of sample to the different lectin molecules is displayed on the solid substrate.

8. The method according to any one of claims 5 to 7, wherein the solid substrate is in a form of a chip.

9. The method according to claim 7 or 8, wherein the cell is **characterized by** analyzing the displayed pattern with cluster analysis.

## Patentansprüche

1. Verfahren zur Analyse von Zuckerketten in einer Probe unter Verwendung einer Lektinbibliothek, wobei die Lektinbibliothek mindestens ein rekombinantes *Maackia amurensis* Hemagglutinin-Lektinmolekül umfasst, bei dem die Kohlenhydratbinderegion eine Aminosäuresequenz, ausgewählt aus der Gruppe, bestehend aus
Asp Thr Tyr Gln Ala His Ile Thr Asp His Ser,
Asp Thr Tyr Leu Pro His Ser Leu Asp Asn Arg,
Asp Thr Tyr Glu Arg His Thr Gly Asp Gly Met,
Asp Thr Tyr Ser Ala His Pro Gly Asp Val Val,
Asp Thr Tyr Ala Leu His Pro Gly Asp Ser Phe,
Asp Thr Tyr Ser Arg His Pro Phe Asp Ser Glu,
Asp Thr Tyr Leu Cys His Ser Leu Asp Trp Leu,
Asp Thr Tyr Gly Gly His Ser Pro Asp Phe Val,
Asp Thr Tyr Ser Ala His Ser Ser Asp Asp Ala, und
Asp Thr Tyr Arg Val His Ala Ser Asp Ala Trp
umfasst und wobei die Zuckerkette ausgewählt ist aus der Gruppe, bestehend aus Glycophorin, Neu5Acα2-3Galβ1-3GalNAcα-, GalNAcα1-3GalNAcβ-, Neu5Acα2-6Gal-NAcα- und 3'Sialyllactosamin-Resten.

2. Verfahren gemäß Anspruch 1, wobei die Lektinmoleküle in der Lektinbibliothek durch Schwenken mit Zellen oder Pseudozellen, die bestimmte Zuckerketten haben, ausgewählt werden.

3. Verfahren gemäß Anspruch 1 oder 2, bei dem Zellen mit Zuckerketten diskriminiert und/oder identifiziert werden.

4. Verfahren gemäß Anspruch 3, wobei die Zellen diskriminiert und/oder identifiziert werden, indem die Bindungen der Zellen mit verschiedenen Lektinmolekülen nach Charakterisieren des Bindungsmusters durch Clusteranalyse verglichen werden.

5. Verfahren gemäß einem der Ansprüche 1 bis 4, wobei die Lektinbibliothek als eine Anordung von Lektinen auf einem festen Substrat gebildet wird und die verschiedenen Lektinmoleküle in der Bibliothek auf vordefinierte Regionen des Substrats festgelegt sind.

6. Verfahren gemäß Anspruch 5, wobei die vordefinierten Bereiche, auf die die verschiedenen Lektinmoleküle festgelegt sind, in einer für das Charakterisieren einer Zelle, die eine Zelloberflächenzuckerkette trägt, geeigneten Reihenfolge angeordnet sind.

7. Verfahren gemäß Anspruch 5 oder 6, wobei das Bindemuster der Probe mit den verschiedenen Lektinmolekülen auf dem festen Substrat dargestellt wird.

8. Verfahren gemäß einem der Ansprüche 5 bis 7, wobei das feste Substrat in Form eines Chips vorliegt.

9. Verfahren gemäß Anspruch 7 oder 8, wobei die Zelle durch Analysieren des dargestellten Musters mit Clusteranalyse charakterisiert wird.

## Revendications

1. Méthode d'analyse de chaînes de sucres dans un échantillon en utilisant une banque de lectines, dans laquelle la banque de lectines comprend au moins une molécule de lectine consistant en hémagglutinine de *Maackia amurensis* recombinante dont la région de liaison aux glucides comprend une séquence d'aminoacides choisie dans le groupe consistant en :
Asp Thr Tyr Gln Ala His Ile Thr Asp His Ser,
Asp Thr Tyr Leu Pro His Ser Leu Asp Asn Arg,
Asp Thr Tyr Glu Arg His Thr Gly Asp Gly Met,
Asp Thr Tyr Ser Ala His Pro Gly Asp Val Val,
Asp Thr Tyr Ala Leu His Pro Gly Asp Ser Phe,
Asp Thr Tyr Ser Arg His Pro Phe Asp Ser Glu,
Asp Thr Tyr Leu Cys His Ser Leu Asp Trp Leu,
Asp Thr Tyr Gly Gly His Ser Pro Asp Phe Val,
Asp Thr Tyr Ser Ala His Ser Ser Asp Asp Ala, et
Asp Thr Tyr Arg Val His Ala Ser Asp Ala Trp
et dans laquelle ladite chaîne de sucres est choisie dans le groupe consistant en des résidus glycophorine, Neu5Acα2-3Galβ1-3GalNacα-, GalNAcα1-3GalNacβ-, Neu5Acα2-6GalNacα-et 3'-sialyllactosamine.

2. Méthode suivant la revendication 1, dans laquelle les molécules de lectine dans ladite banque de lectines sont choisies par dépistage avec des cellules ou pseudo-cellules ayant des chaînes de sucres prédéterminées.

3. Méthode suivant la revendication 1 ou 2, par laquelle les cellules ayant des chaînes de sucres sont différenciées et/ou identifiées.

4. Méthode suivant la revendication 3, dans laquelle les cellules sont différenciées et/ou identifiées en comparant les liaisons des cellules à des molécules de lectines différentes après caractérisation du motif de liaison par analyse par groupes.

5. Méthode suivant l'une quelconque des revendications 1 à 4, dans laquelle la banque de lectines est formée à l'état d'un réseau de lectines sur un substrat solide et les différentes molécules de lectines dans ladite banque sont immobilisées à des régions prédéfinies du substrat.

6. Méthode suivant la revendication 5, dans laquelle les régions prédéfinies auxquelles les différentes molécules de lectines sont immobilisées sont disposées dans un ordre convenable pour la caractérisation d'une cellule portant une chaîne de sucres de surface cellulaire.

7. Méthode suivant la revendication 5 ou 6, dans laquelle le motif de liaison de l'échantillon aux différentes molécules de lectines est représenté sur le substrat solide.

8. Méthode suivant l'une quelconque des revendications 5 à 7, dans laquelle le substrat solide est sous forme d'une puce.

9. Méthode suivant la revendication 7 ou 8, dans laquelle la cellule est **caractérisée en** analysant le motif représenté par analyse par groupes.
